(19) Europäisches Patentamt
European Patent Office
Office européen des brevets

(11) EP 1 816 187 A1

(12) EUROPEAN PATENT APPLICATION
published in accordance with Art. 158(3) EPC

(43) Date of publication:
08.08.2007 Bulletin 2007/32

(51) Int Cl.:
C12M 1/00 (2006.01)          B01J 19/00 (2006.01)
B81B 1/00 (2006.01)          C12M 1/34 (2006.01)
G01N 37/00 (2006.01)

(21) Application number: 05806585.5

(22) Date of filing: 18.11.2005

(86) International application number:
PCT/JP2005/021231

(87) International publication number:
WO 2006/054689 (26.05.2006 Gazette 2006/21)

(84) Designated Contracting States:
CH DE FR GB LI SE

(30) Priority: 22.11.2004 JP 2004338002

(71) Applicants:
• NISSUI PHARMACEUTICAL CO., LTD.
Taito-ku,
Tokyo 110-8736 (JP)
• KANAGAWA ACADEMY OF SCIENCE AND
TECHNOLOGY
Kawasaki-shi,
Kanagawa-ken 213-0012 (JP)

(72) Inventors:
• Akaba, Shuichi,
Nissui Pharmaceutical Co. Ltd.
Yuuki-shi,
Ibaraki 3070036 (JP)

• Oku, Yuichi,
Nissui Pharmaceutical Co. Ltd.
Yuuki-shi,
Ibaraki 3070036 (JP)
• Tokeshi, Manabu
Takatsu-ku,
Kanagawa 2130012 (JP)
• KITAMORI, Takehiko
1130033 (JP)

(74) Representative: Fiener, Josef et al
Patentanw. J. Fiener et col.,
P.O. Box 12 49
87712 Mindelheim (DE)

(54) MICROCHIP

(57)     A microchip used for a diagnosis device having a micro fluid system, reaction efficiency of which microchip can be drastically increased and which has a flow path realizing measurement with high stability and reproducibility. A microchip in which at least a flow path (12) is formed in interfaces of two substrates and that has, in the flow path (12), a reaction region (14) and a detection region (15) on the downstream side of the reaction region (14). The depth of the flow path (12) in the detection region (15) is greater than the depth of the flow path (12) in the reaction region (14).

Fig.2

**Description**

**TECHNICAL FIELD**

This invention relates to a microchip capable of remarkably improving the reaction efficiency which has a flow path capable of conducting a stable measurement with high reproducibility. More specifically, this invention relates to a microchip adapted for a high sensitivity diagnosis.

**BACKGROUND ART**

Recently, chemical assay systems called μTAS (micro total analysis system) or "lab-on-a-chip" are eagerly developed. This is a chemical assay system (hereinafter sometimes referred to as μTAS) carried out by using the so called "microchip" which is a glass, silicon or plastic substrate of several centimeters square formed with a minute flow path having a cross-sectional width of several micrometers to several millimeters by which the chemical assay procedures are integrated. This system is known to have various merits including increase in the efficiency of the chemical reaction and remarkable reduction in the reaction time which is realized by the increase of the specific interface area. Specific interface area may be represented as "the area of the solid-liquid interface in relation to the liquid volume", and in the case of the microchip formed with a minute flow path, concretely, the specific interface area may be represented as "the surface area of the walls of the flow path in relation to the solution volume". The specific interface area can be increased by employing a microchip formed with a minute flow path of several micrometers to several millimeters.
In the case of μTAS, the reaction time is also remarkably reduced due to the smaller space of the flow path in the microchip which in turn means the shorter diffusion length of the substance.

Immunoassay systems in which μTAS has been used in a diagnostic assay system have been reported. In the construction of an immunoassay system using μTAS, an antibody should be placed in the flow path by certain means for capturing the target substance to be measured. Sato et al. (Sato et al., Analytical chemistry 2001, 73, 1213-1218) (Non-patent Document 1) reports the detection by forming a dam structure in the flow path of the microchip, filling polystyrene beads having an antibody bonded thereto in the upstream of the dam structure, supplying the sample, and then, an labeled antibody to the flow path to thereby form an antigen-antibody complex on the surface of the polystyrene beads; and detecting the labeled substance of the antigen-antibody complex by thermal lens microscope. The immunoassay system of the Non-patent Document 1 has been accomplished by integrating the immunoassay system which is generally carried out on a microtiter plate or the like on a minute space on a microchip, and Non-patent Document 1 reports that remarkable reduction in the reaction time and improvement of the detection sensitivity have been realized compared to the conventional system using a microtiter plate.

When sensitivity is to be improved in the immunoassay system of Non-patent Document 1 using the microchip provided with a flow path having a dam structure to hold the polystyrene beads, the beads should be filled in the flow path at a high density if the reaction area were to be increased. In this case, the solution should be supplied at an increased pressure, and accordingly, accurate control of the flow rate as well as uniform supply of the solution to the space defined between the beads would be difficult.

Lab on a chip, 2002, 2, 27-30 (Non-patent Document 2) discloses an assay system in which electrochemical detection is carried out by immobilizing an antibody on the surface of magnetic particles, immobilizing the thus prepared magnetic particles on the magnet provided in the micro-flow path by feeding a solution containing the magnetic particles, and supplying the sample, and then the labeled antibody to the flow path to thereby form an immune complex. The method of Non-patent Document 2 had the merit that the reaction time is reduced compared to the conventional microtiter plate method widely used in the art. However, improvement in the detection sensitivity was not accomplished by this method, probably because of the excessive distance between the surface of the magnetic particles on which the antigen-antibody complex had formed and the electrochemical sensor provided for the detection, which resulted in the weak electrochemical signal reaching the sensor. This situation may be improved to some extent by using a shallower flow path, but there should be a technological limitation in such improvement.

Biosensors and Bioelectronics 19 (2004) 1193-1202 (Non-patent Document 3) discloses a micro mosaic immunoassay as an example of an assay system measuring multiple items at once. In the Non-patent Document 3, the detection is accomplished by directly immobilizing an antibody on a substrate, supplying the sample, and then the fluorescence-labeled antibody to the flow path for formation of an immune complex on the substrate, and detecting the labeled substance by fluorescence microscopy. However, superiority has not been recognized for the method of the Non-patent Document 3 over the latex turbidimetry which is a popular immunoassay method in the immunoassay. In addition, in the case of the microchip of the Non-patent Document 3, the substrate on which the antibody is to be immobilized and the substrate formed with the flow path are both formed from tacky polydimethylsiloxane, and while the substrates can be adhered without heat sealing, this microchip is less adequate as a commercial microchip product which is required to have a high level detection accuracy since the substrates are made of a rubber material with unstable shape retainability.

Non-patent Document 1: Sato et al., Analytical chemistry 2001, 73, 1213-1218.
Non-patent Document 2: Lab on a chip 2002, 2, 27-30.
Non-patent Document 3: Biosensors and Bioelectronics 19(2004) 1193-1202.

## DISCLOSURE OF THE INVENTION

## PROBLEMS TO BE SOLVED BY THE INVENTION

In the case of μTAS having the minute reaction field, the technology used in detecting the signal produced by the reaction is limited by the minuteness of the reaction field. For example, in the μTAS attempting to realize the stable detection with high sensitivity, the signal obtained in the reaction area is amplified, and the amplified signal is detected after its transfer to the detection area which is separately provided from the reaction area. When a microchip in which the reaction efficiency has been improved by simply reducing the volume of the entire flow path is used in such a case, the size of the detection area will also be reduced as a matter of course, and the thus reduced optical path length results in the difficulty of attaining detection reproducibility.

Difficulty of realizing the reproducibility in μTAS is further described by referring to the case of immunoassay system. When an enzyme such as peroxidase, alkaline phosphatase, or glucose oxidase is used for labeling an antibody (ELISA), a substrate for the enzyme may be supplied to measure the substrate which has undergone the enzymatic reaction by detecting the label in the detection area provided in the downstream of the reaction area. The substrates which may be used include a chromogenic substrate, a fluorescent substrate, and a chemiluminescent substrate. In this case, increase in the detection sensitivity and stability of the detection signal can be expected only when the detection area in the microfluid system used for detecting such substrate which has undergone the enzymatic reaction has certain size.

For example, when the detection is conducted by using a fluorescent substrate or a chemiluminescent substrate, the detection sensitivity increases when the metabolite of the substrate generated by the enzymatic reaction is present in the detection area at a larger absolute amount.

When a chromogenic substrate is detected by using a thermal lens microscope by focusing a excitation beam at a position in the flow path, a higher reproducibility in the signal detection is realized at a longer optical path length, and when the optical path length is below a certain value, the signal is reduced to detract from the signal detection reproducibility. More specifically, thermal lens microscope is associated with errors caused by factors such as mechanical vibration, insufficient accuracy of the positioning in the detection, insufficient precision in the production of the microchip, and such errors inevitably results in the change in the relative position of the focal point in the flow path. When the error is larger than the optical path length, for example, when the optical path length is excessively short that the error exceeds the optical path length, the excitation beam of the thermal lens microscope may be focused at a point outside the flow path, and in such a case, the measurement can not be carried out. In contrast, when the flow path has a sufficiently long optical path length, stable reproducibility can be realized since deviation of the focal length within certain extent can be deemed as relative change in the position of the focal point within the flow path. When the excitation beam is irradiated from upper side of the flow path (from upper surface of the plate-shaped microchip), depth of the flow path as seen from the direction of the irradiation source corresponds to the optical path length, whereas the width of the flow path is deemed the optical path length when the beam is irradiated in the side direction of the flow path (from the side surface of the plate-shaped microchip).

However, the technology for conducting the detection in such a minute space is still under development, and even if such detection were possible in the research and development phase, realization of the high detection sensitivity and the stable and reproducible detection signal required for an assay system as typically used in clinical test, environmental test, and food test is difficult.

An object of the present invention is to provide a microchip which is used in a diagnostic system using a microfluid system, and which has a flow path capable of greatly improving the reaction efficiency and realizing a stable measurement with high reproducibility.

## MEANS TO SOLVE THE PROBLEMS

In order to realize such object, this invention provides a first microchip. In this first microchip, the microchip comprises two substrates having at least a flow path formed at the interface between the two substrates, and the flow path has a reaction area and a detection area in the downstream of the reaction area. The flow path at the detection area has a depth which is deeper than the flow path at the reaction area.

This invention also provides a second microchip. In this first microchip, the microchip comprises two substrates having at least a flow path formed at the interface between the two substrates, and the flow path has a reaction area and a detection area in the downstream of the reaction area. The flow path at the detection area has a width which is wider than the flow path at the reaction area, and the flow path at the detection area has a depth which is deeper than the flow path at the reaction area.

In the present invention, "depth of the flow path" is length of the flow path in the direction the same as the direction of detection. For example, in the case of a plate-shaped microchip which is detected in the direction perpendicular to the plane of the plate, the depth of the flow path is the inner size of the flow path in the direction of the detection which is the direction perpendicular to the plane of the plate. When the detection is conducted from the side surface, the depth of the flow path is the inner size of the flow path in the direction of the detection which is the direction perpendicular to the side surface.

In the present invention, the "width of the flow path" is the inner size of the flow path in the direction perpendicular to the direction of the detection.

In the present invention, the "direction of the detection" is, in the case of the detection by thermal lens microscope or the detection by the fluorescence, the direction of the incidence of the excitation beam into the flow path. In the case of the detection by absorption, it is the direction of the incidence of the light beam having a wavelength absorbed by the substance to be measure; and in the case of the detection by luminescence, it is the direction of the incidence of the luminescent beam which is detected by the detector which detects the luminescence.

In the present invention, the "reaction area" has an interface which contributes for the reaction, and this interface is at least a part of the interface of the flow path, namely a part or all of the interface in the flow path. Preferably, the reaction area has, for example, an anti-biological substance immobilized on the inner surface of the flow path. This anti-biological substance is capable of binding to the biological substance to be measured by affinity to thereby capture the biological substance.

## EFFECTS OF THE INVENTION

(i) In the first microchip of the present invention, the flow path in the detection area is deeper than the flow path in the reaction area, and therefore, the microchip has high detection stability and reproducibility. At the same time, since the flow path in the reaction area is relatively shallow compared to the flow path in the detection area, the flow path in the reaction area has a greater specific interface area, and reaction efficiency in the reaction area is improved to realize the high sensitivity.

(ii) In the second microchip of the present invention, the flow path in the reaction area is wider than the flow path in the detection area, and the flow path in the reaction area is shallower than the flow path in the detection area, and therefore, the flow path in the reaction area has a greater specific interface area, and reaction efficiency in the reaction area is improved to realize the high sensitivity, and at the same time, since the flow path in the detection area is relatively deep compared to the flow path in the reaction area, the microchip has higher detection stability and reproducibility.

(iii) In the microchip of the present invention, an anti-biological substance which is capable of binding to the biological substance by affinity is immobilized on the interface of the flow path in the reaction area in order to capture the biological substance (the substance to be measured), and therefore, the microchip has the merit that it is highly useful as a diagnostic microchip capable of assaying a biological substance in addition to the merits as described above in (i) and (ii).

## BRIEF DESCRIPTION OF THE DRAWINGS

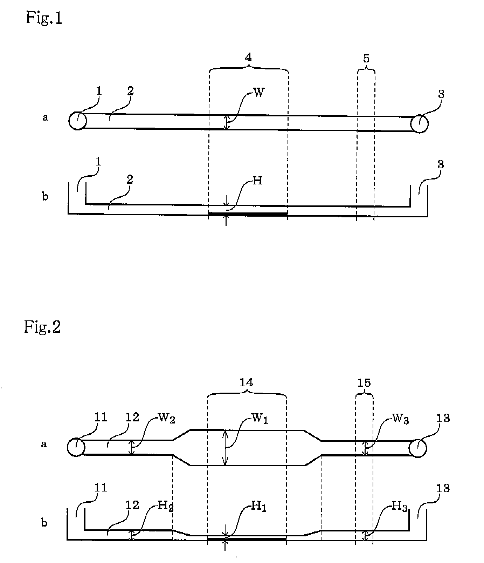

[FIG. 1] FIG. 1 shows the standard-type flow path which was prepared as a contrast. In this flow path, the reaction area and the areas in the upstream and the downstream of the reaction area have the same cross-sectional shape (with a width of 0.3 mm and a depth of 0.1 mm). FIG. 1a is a top view of the flow path, and FIG. 1b is a cross-sectional view of the flow path.

[FIG. 2] FIG. 2 shows an embodiment of the flow path according to the present invention. FIG. 2a is a top view of the flow path, and FIG. 2b is a cross-sectional view of the flow path.

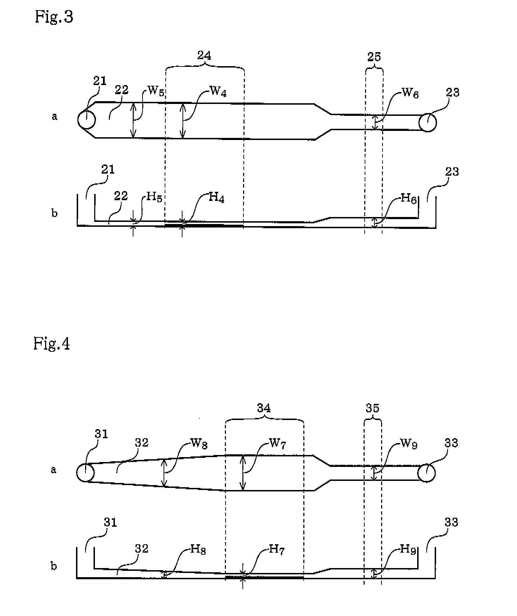

[FIG. 3] FIG. 3 shows another embodiment of the flow path in the microchip of the present invention. FIG. 3a is a top view of the flow path, and FIG. 3b is a cross-sectional view of the flow path.

[FIG. 4] FIG. 4 shows a further embodiment of the flow path in the microchip of the present invention. FIG. 4a is a top view of the flow path, and FIG. 4b is a cross-sectional view of the flow path.

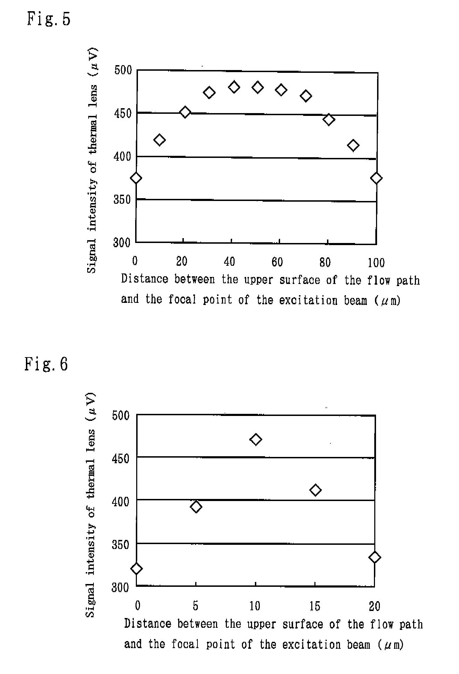

[FIG. 5] FIG. 5 is a graph showing signal intensity of the thermal lens microscope at different focal points of the excitation beam when the flow path has a depth of 0.1 mm. Y axis represents signal of the thermal lens, and X axis represents distance between the focal point of the excitation beam and the upper surface of the flow path.

[FIG. 6] FIG. 6 is a graph showing signal intensity of the thermal lens microscope at different focal points of the excitation beam when the flow path has a depth of 0.02 mm. Y axis represents signal of the thermal lens, and X axis represents distance between the focal point of the excitation beam and the upper surface of the flow path.

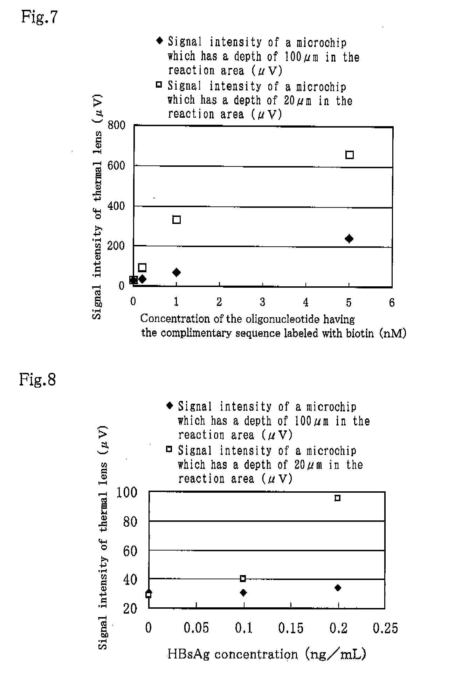

[FIG. 7] In Example 2, signal in the depth direction of the flow path was detected by thermal lens microscope in the detection area in the downstream of the reaction area while supplying SATBlue (product name, manufactured by Dojindo Laboratories). The detection was conducted by adjusting the excitation beam to a wavelength of 633 nm and the probe beam to a wavelength of 488 nm. The results are shown in the graph wherein the Y axis represents signal intensity of the thermal lens and the X axis represents concentration of the biotin-labeled oligonucleotide having the complementary sequence.

[FIG. 8] In Example 3, signal in the depth direction of the flow path was detected by thermal lens microscope in the detection area in the downstream of the reaction area while supplying SATBlue (product name, manufactured by Dojindo Laboratories). The detection was conducted by adjusting the excitation beam to a wavelength of 633 nm and the probe beam to a wavelength of 488 nm. The results are shown in the graph of FIG. 7 wherein the Y axis represents signal intensity of the thermal lens and the X axis represents concentration of the HBsAg.

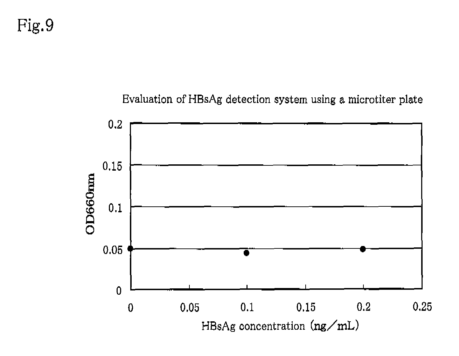

[FIG. 9] In Comparative Example 1, color development of SATBlue (product name, manufactured by Dojindo Laboratories) was measured by the absorption at 660 nm. The results are shown in the graph wherein the Y axis represents the absorbance, and the X axis represents concentration of the HBsAg.

**LEGEND**

1, 11, 21, 31 Inlet
2, 12, 22, 32 Flow path
3, 13, 23, 33 Outlet
4, 14, 24, 34 Reaction area
5, 15, 25, 35 Detection area

**BEST MODE FOR CARRYING OUT THE INVENTION**

In the microchip of the present invention, the reaction area in the flow path has a specific interface area larger than that of the detection area in the flow path. In other words, the reaction area has a volume which is smaller than that of the detection area in the flow path, and accordingly, reaction efficiency in the reaction area increases by the increase in the diffusion efficiency of the substance to the interface.

The reaction area of the microchip of the present invention has an interface which contributes for the reaction, and this interface constitutes at least a part, namely, a part or all of the interface of the flow path.

The flow path in the microchip of the present invention may have a cross-section of various shapes including triangle, square, rectangle, trapezoid, parallelogram, other quadrangles, polygons having 5 or more apexes, circle, oblong and other shapes in which case the entire interface is curved, shapes such as semicircle in which case at least one part of the interface is a flat surface and other parts are curved.

With regard to the reactivity at the interface of the flow path, decrease in the volume of the flow path results in the increase of the specific interface area, and hence, increase in the diffusion efficiency of the substance to the interface results in the improved reaction efficiency. For example, in the case of the detection using a flow path having a rectangular cross-section and conducing the detection from the upper surface of the flow path to the lower surface of the flow path, when the lower surface or the upper surface or both surface of the flow path are the interface contributing for the reaction, reduction in the depth of the flow path results in the increase of the specific interface area of the reaction, and hence, increase in the reactivity. When one or both of the side surfaces of the flow path is the reaction surface, reduction in the width of the flow path results in the increase of the specific interface area, and hence, increase in the reactivity. When the entire surface of the flow path is the reaction surface, reduction of both or either one of the width and the depth of the flow path results in the increase in the specific interface area, and hence, increase in the reactivity. Accordingly, the volume of the reaction area is preferably reduced to the minimal level that enables stable transfer of the liquid.

In this case, "lower surface of the flow path" means the bottom surface of the flow path when the plate-shaped microchip is placed in a horizontal position.

In this case, "upper surface of the flow path" means the upper surface of the flow path when the plate-shaped microchip is placed in a horizontal position.

In this case, "side surface of the flow path" means the interior surface of the flow path which is parallel to the side surfaces of the microchip.

In the present invention, "specific interface area" means ratio of the surface area of the walls of the flow path to the volume of the flow path. For example, in the case of a flow path having a rectangular cross-section having a width of 0.1 mm, a depth of 0.2 mm, and a length of 0.5 mm, the specific interface area is calculated by the following equation to be 30.

```
        (Surface area of the flow path walls) / (volume of
    the flow path)
        =  {(0.1 mm x 0.5 mm x 2)+(0.2 mm x 0.5 mm x 2)} /
    (0.1 mm x 0.2 mm x 0.5 mm)
        = 30
```

For the bottom surface (width 0.1 mm, length 0.5 mm) of the flow path, the specific interface area is calculated by the following equation to be 5.

```
        (Surface area of the bottom surface of the flow
    path) / (volume of the flow path) = (0.1 mm x 0.5 mm) /
    (0.1 mm x 0.2 mm x 0.5 mm) = 5
```

When the depth of the flow path is reduced from 0.2 mm to 0.1 mm, the specific interface area of the bottom surface of the flow path is doubled to 10 as calculated by the following equation.

```
        (Surface area of the bottom surface of the flow
    path) / (volume of the flow path) = (0.1 mm x 0.5 mm) /
    (0.1 mm x 0.1 mm x 0.5 mm) = 10
```

In the present invention, "linear flow velocity" is the distance which the liquid advances in predetermined unit time, and this "linear flow velocity" is a concept different from the "flow rate" which is the volume of the liquid transferred per unit time. For example, when the liquid is supplied at the same flow rate to a cylindrical flow path A having a rectangular cross-section with a width of 0.1 mm and a depth of 0.2 mm and a cylindrical flow path B having a square cross section with a width of 0.1 mm and a depth of 0.1 mm, the distance at which the liquid advances at a particular time in the case of the flow path B will be twice that of the flow path A, because the flow path A has a depth twice larger than that of the flow path B and hence, a volume twice larger than that of the flow path B. As described above, the linear flow velocity of the flow path B is twice that of the linear flow velocity of the flow path A even though both flow paths have the same flow rate.

FIGS. 2a and 2b show an embodiment of the flow path in the microchip of the present invention. FIG. 2a is a top view of the flow path, and FIG. 2b is a cross-section of the flow path. A reaction area 14 is provided in the middle of the flow path 12, and in this reaction area 14, an anti-biological substance is immobilized in order to capture the biological substance to be measured. An inlet 11 is provided at one end of the flow path 12 for supplying the test reagent and the sample, and an outlet 13 is provided at the other end of the flow path 12 for discharging the liquid in the flow path 12. In the flow path 12 in the downstream of the reaction area 14, a detection area 15 is provided to measure the signal in the flow path 12 by using an analytical means such as fluorescence, chemiluminescence, and thermal lens spectroscopy. Width $W_1$, of the flow path in the reaction area 14 is larger than widths $W_2$ and $W_3$ in the upstream and downstream of the reaction area 14, and depth $H_1$ of the flow path in the reaction area 14 is smaller than depths $H_2$ and $H_3$ in the upstream and downstream of the reaction area 14.

FIGS. 3a and 3b show another embodiment of the flow path in the microchip of the present invention. FIG. 3a is a top view of the flow path, and FIG. 3b is a cross-section of the flow path. A reaction area 24 is provided in the flow path 22, and in this reaction area 24, an anti-biological substance is immobilized in order to capture the biological substance to be measured. An inlet 21 is provided at one end of the flow path 22 for supplying the test reagent and the sample, and an outlet 23 is provided at the other end of the flow path for discharging the liquid in the flow path 22. In the flow path 22 in the downstream of the reaction area 24, a detection area 25 is provided to measure the signal in the flow path 22 by using an analytical means such as fluorescence, chemiluminescence, and thermal lens spectroscopy. Width $W_4$ of the flow path in the reaction area 24 is larger than width $W_6$ of the detection area 25 and the same as width $W_5$ of the flow path in the upstream of the reaction area 24. Depth $H_4$ of the flow path in the reaction area 24 is smaller than the depth $H_6$ in the upstream and downstream of the reaction area 24, and the same as the depth $H_5$ in the upstream of the reaction area 24.

FIGS. 4a and 4b show a further embodiment of the flow path in the microchip of the present invention. FIG. 4a is a top view of the flow path, and FIG. 4b is a cross-section of the flow path. A reaction area is provided in the middle of the flow path 32, and in this reaction area 34, an anti-biological substance is immobilized in order to capture the biological substance to be measured. An inlet 31 is provided on one end of the flow path 32 for supplying the test reagent and the sample, and an outlet 33 is provided on the other end of the flow path 32 for discharging the liquid in the flow path. In the flow path 32 in the downstream of the reaction area 34, a detection area 35 is provided to measure the signal in the flow path 32 by using an analytical means such as fluorescence, chemiluminescence, and thermal lens spectroscopy. Width $W_7$ of the flow path in the reaction area 34 is larger than widths $W_8$ and $W_9$ in the upstream and downstream of the reaction area 34, and depth $H_7$ of the flow path in the reaction area 34 in is smaller than depths $H_8$ and $H_9$ in the upstream and downstream of the reaction area 34. The width $W_8$ of the flow path 32 in the upstream of the reaction area 34 gradually increases toward the downstream with the gradual decrease in the depth He. By such gradual change in the shape of the flow path 32, linear flow velocity in the flow path 32 can be controlled to a constant value or to gradually increase or decreases as desired.

With regard to the flow of the sample or the test reagent in the microchip, when the flow rate to adopt is matched with the flow rate of reaction area, the flow rate of the solution after passing through the reaction area will be low, and an excessive time will be required before reaching the detection area detracting from the requirement of quick assay. In the opposite case, the solution will almost instantly pass through the reaction area without realizing the merit of increasing the width, and realization of the intended signal amplification may become difficult. In order to obviate such inconvenience, the flow path may be designed so that width of the flow path in the reaction area increases with the decrease of the depth of the corresponding part, and the depth of the flow path in the reaction area increases with the decrease in the width of the corresponding part to thereby increase the specific interface area of the reaction surface in the reaction area while controlling the linear flow velocity of the overall flow path to a constant level.

Also, the flow path may be designed so that the solution passes through the reaction area at an intentionally increased or reduced linear flow velocity. For example, when the depth of the flow path in the reaction area is reduced by half, the width of the flow path should be doubled to keep the linear flow velocity at the same level. However, when the width of the flow path is increased 4 times, the linear flow velocity in the reaction area will be half. When the flow path is designed in such way, amplification efficiency will be doubled with the increase in the assay time. As exemplified by this embodiment, linear flow velocity in the reaction area can be controlled depending on the intended situation.

In the microchip of the present invention, the width of the flow path in the reaction area is preferably at least 1 $\mu$m and up to 2 mm, and more preferably at least 1$\mu$ m and up to 500 $\mu$m. When the width of the flow path is less than 1 $\mu$m, resistance caused by the surface tension of the solution will be increased, and stable supply of the solution is difficult since an extremely high pressure is required for the supply of the solution into the flow path. On the other hand, width of the flow path in excess of 2 mm results in the turbulence of the solution and uniform supply of the solution into the flow path will be difficult.

In the microchip of the present invention, depth of the flow path in the reaction area is preferably at least 1 $\mu$m and up to 2 mm, and more preferably, at least 1 $\mu$m and up to 500 $\mu$m. When the depth of the flow path is less than 1 $\mu$m, resistance caused by the surface tension of the solution will be increased, and stable supply of the solution is difficult since an extremely high pressure is required for the supply of the solution into the flow path. On the other hand, depth of the flow path in excess of 2 mm results in the turbulence of the solution and uniform supply of the solution into the flow path will be difficult.

In the microchip of the present invention, depth of the flow path in the detection area is preferably at least 10 $\mu$m and up to 2 mm. When the depth of the flow path is less than 10 $\mu$m, assay by the microchip will be difficult. On the other hand, depth of the flow path in excess of 2 mm results in the turbulence of the solution and uniform supply of the solution into the flow path will be difficult.

The two substrates used in producing the microchip of the present invention may be made of polydimethylsiloxane (abbreviation PDMS, Anal. Chem., Vol.69, pp. 3451-3457, 1997), acrylic resin (Anal. Chem., Vol.69, pp. 2626, 1997), polymethyl methacrylate (abbreviation: PMMA, Anal. Chem., Vol.69, pp. 4783, 1997), glass, cyclic olefin polymer, or such material surface modified with diamond or diamond-like carbon (Japanese Patent Application Laid-Open No. 2002-365293), cetyltrimethyl ammonium bromide(CTAB), Surmodics, Reacti-Bind (Analytical Biochemistry, 317 (2003) 76-84), poly-L-lysine, carbodiimde, amino group, aldehyde group, meleimide group, or dextrane. While the substrate may be either transparent or non-transparent, at least the detection area is preferably transparent when a non-transparent substrate is used. In addition, the substrate may be subjected to an optional hydrophilic or a hydrophobic treatment.

The microchip of the present invention can be produced, for example, by preparing a mold by etching a silicon wafer, and introducing a molten polymer into the mold to transfer the structure of the mold and allowing the polymer to set. In this procedure, one of the substrate, namely, the substrate having the groove (which will be the flow path in the finished product) is formed. The microchip is completed when this substrate is adhered with the other plate-shaped substrate.

Adhesion of the two substrates is generally accomplished by heating. When the substrate is produced from PDMS, sealing of the flow path can be accomplished in simple way by natural adhesion between the glass or PDMS. Microchips made from a plastic material is well adapted for mass scale production, and therefore, economically advantageous. While the depth is adjusted in the case of the glass substrate by the time of reaction with hydrogen fluoride, a plastic substrate can be produced with high reproducibility by the injection molding technique once the mold is produced. The two substrate is typically adhered by using an adhesive or by heat sealing. Exemplary adhesives include organic adhesives such as adhesives ionizing radiation curable adhesives (such as UV curable adhesives), thermosetting adhesives, acrylic adhesives, and epoxy adhesives, and inorganic adhesives.

The substance detected by the microchip of the present invention is preferably a biological substance. Examples of such biological substance include an antigen, antibody, sugar chain, glycoprotein, lectin, receptor, ligand, DNA, RNA, and other substance which is capable of specifically binding to a particular substance in a living body irrespective of the molecular weight of the biological substance. The specimens which can be used in detecting such biological substance include body fluids such as blood, plasma, serum, urine, and saliva, and specimens containing DNA, RNA, chromosome, an amplification product of DNA or RNA, antigen, antibody, sugar chain, receptor, or ligand.

Examples of the anti-biological substance immobilized on the interface of the reaction area which contributes for the reaction of the microchip of the present invention include an antibody (against an antigen), an antigen (against an antibody), avidin (against biotin), biotin (against avidin), a DNA (against a DNA), a DNA (against an RNA), a ligand (against a receptor), and a receptor (against a ligand).

The anti-biological substance may be immobilized on the interface of the reaction area either by directly bonding the anti-biological substance to the interface or by indirectly bonding the anti-biological substance to the interface using an intervening ligand. Exemplary methods used for the direct bonding of the anti-biological substance to the interface of the reaction area include use of ionic bond, hydrogen bond, or hydrophobic bond, and immobilization of the anti-biological substance by covalent bond to the chemically modified interface.

Exemplary intervening ligands which may be used in binding the anti-biological substance to the interface of the reaction area include nucleic acids such as deoxyribonucleic acid which is stable at elevated temperature or in the presence of an organic solvents. Bonding of the anti-biological substance can be accomplished by preliminarily immobilizing the nucleic acid used for the ligand on the interface of the reaction area, thermally adhering the substrate having the nucleic acid immobilized and the substrate formed with the groove which will be the flow path, and then, supplying a solution containing the anti-biological substance having a nucleic acid having a sequence which is complementary to the nucleic acid immobilized on the substrate thereto to the flow path to thereby immobilize the anti-biological substance on the interface of the reaction area. In the case of the microchip produced by such procedure, the only biological component that is exposed to heat is the nucleic acid which has a relatively high resistance to heat, and heating of the anti-biological substance can be avoided. Accordingly, denaturing of the thermally sensitive anti-biological substance such as a protein is avoided and the anti-biological substance retains its activity. When the substance to be detected is a nucleic acid, a nucleic acid having the sequence complementary to the nucleic acid to be detected may be immobilized on the interface of the reaction area.

In addition to such nucleic acid, magnetic particles may also be used as a ligand for immobilizing the anti-biological substance to the interface of the reaction area. In this case, the anti-biological substance can be immobilized on the interface of the reaction area by preliminarily providing a magnet inside or outside the flow path of the reaction area, and supplying a solution containing magnetic particles having the biological substance immobilized on their surface into the flow path of the microchip.

The microchip of the present invention is designed so that at least one interface in the flow path in the reaction area has reactivity. In the thus designed microchip of the present invention, the uniform flow rate and the homogeneous solution are not disturbed as in the case of the conventional microchip having the flow path blocked by polystyrene beads since the microchip of the present invention does not use the polystyrene beads which may block the fluid flow.

In the present invention, detection in the detection area may be accomplished by analytical means such as fluorescence, chemiluminescence, thermal lens spectroscopy, or absorption spectroscopy. A stable detection with high reproducibility has been realized by the microchip of the present invention since the flow path in the detection area has a depth greater than the flow path in the reaction area. For example, in the case of the thermal lens spectroscopy, the greater depth of the flow path in the detection area of the present microchip contributes for the improved detection stability and reproducibility since focusing of the excitation beam to a point within the flow path is facilitated, and in particular, because the signal intensity does not significantly change as long as the excitation beam is focused at a point within few dozen micrometers (in the depth direction of the flow path) from the targeted point near the center of the optical path.

With regard to the measurement using fluorescence, when the flow path is irradiated by the excitation beam that has been focused to the flow path, focusing of the excitation beam to a point within the flow path is facilitated, and this contributes for the improved stability and reproducibility of the detection. When the measurement is conducted by irradiating the flow path with a non-focused excitation beam, increased absolute amount of the excited fluorescent substance contributes for the improved detection sensitivity.

In the case of the measurement using chemiluminescence, increased absolute amount of the luminescent substance contributes for the improvement of the detection sensitivity.

In each of the optical measurements as described above, depth of the flow path in the detection area will be equal to the optical path length when the detection beam is irradiated across the flow path of the microchip in the direction perpendicular to the axial direction of the flow path. In other words, the term "optical path length" used in the present invention is the length of the beam within the flow path of the beam passing across the flow path.

**Example 1**

Preparation of microchip

(1) Immobilization of DNA

An oligonucleotide having amino group introduced at its 5' end and having the sequence of 5'-TTGCTAACCCAGAACACTAT-3' was synthesized. This oligonucleotide was immobilized on GeneSlide (product name, manufactured by Nihon Parkerizing Co.,Ltd.) at positions corresponding to the reaction area along the flow path according to the instruction of the manufacturer.

(2) Preparation of flow path and preparation of microchip

Two plates each formed with a groove were prepared by using polydimethylsiloxane (PDMS) plates having a thickness of 1 mm. One plate was formed with a groove corresponding to the standard-type flow path as described in (i), below as a contrast. The other plate was formed with a groove corresponding to the flow path of the present invention as described in (ii), below. The grooves were formed according to the method described in McDonald, J. C. et al., Electrophoresis, Vol. 21, No. 1, 2000, pp. 27-40.

The two types of plates each formed with the groove were respectively adhered to the slide glass having the oligonucleotide immobilized thereon produced in the step (1) as described above to thereby produce the contrast microchip and the microchip of the present invention (Example 1) both having a flow path defined between the plate and the slide glass.

(i) Standard-type flow path (contrast)

FIGS. 1a and 1b are views showing the standard-type flow path which was prepared as contrast. In this standard-type flow path, the DNA-immobilized area (the reaction area 4) and the areas in the upstream and downstream of the DNA-immobilized area (the reaction area 4) have the same cross-sectional shape. FIG. 1a is a top view of the flow path 2, and FIG. 1b is a cross-sectional view of the flow path. The flow path 2 formed has a width of 0.3 mm and a depth of 0.1 mm. An inlet 1 is formed at one end of the flow path, and an outlet 3 is formed at the other end of the flow path for discharging the fluid in the flow path 2. A reaction area 4 is defined in the intermediate area of the flow path 2, and this reaction area 4 has the oligonucleotide immobilized thereon to thereby capture the biological substance. A detection area 5 is provided in the downstream of the reaction area 4 for ' detecting the captured biological substance.

(ii) Embodiment of the flow path according to the present invention

The microchip having the flow path used in Example 1 is the microchip having the flow path with the shape of FIGS. 2a and 2b as described above. In this flow path according to Example 1, the intermediate area in the flow path 12 is the reaction area 14 having the DNA immobilized, and this area as a width of 1.5 mm and a depth of 0.02 mm. The flow path 12 in the upstream and the downstream of the reaction area 14 has a width of 0.3 mm and a depth of 0.1 mm.

Confirmation of signal stability in the detection area having sufficient optical path length

The microchip produced in the production step as described above having a flow path (with a width of 1.5 mm and a depth of 0.02 mm in the reaction area and a width of 0.3 mm and a depth of 0.1 mm in the detection area) was placed in the optical path so that the optical path of the excitation beam passed through the reaction area from upper surface to the bottom surface of the flow path. In the meanwhile, the microchip was also placed in the optical path so that the optical path of the excitation beam passed through the detection area of the flow path of Example 1 from upper surface to the bottom surface. With regard to the reaction area, the focal point of the excitation beam was incrementally moved at a pitch of 5 μm from the upper surface to the lower surface of the flow path, while the focal point of the excitation beam was incrementally moved at a pitch of 10 μm from the upper surface to the lower surface of the flow path for the detection area. The signal intensity at each focal point was measured by a thermal lens microscope. Each flow path was filled with Sunset Yellow (product name, manufactured by Wako Pure Chemical Industries, Ltd.) adjusted with water to $10^{-4}$M for measurement by the thermal lens microscope. The thermal lens microscope was used with the excitation beam at a wavelength of 532 nm which is the absorption wavelength of the Sunset Yellow (product name, manufactured by Wako Pure Chemical Industries, Ltd.) and the probe beam at a wavelength of 633 nm at which absorption by the Sunset Yellow (product name, manufactured by Wako Pure Chemical Industries, Ltd.) was low.

The measurements are shown in FIG. 5 (the detection area with a flow path depth of 0.1 mm) and FIG. 6 (the reaction area with a flow path depth of 0.02 mm) in which the Y axis represents signal intensity of the thermal lens and the X axis represents distance of the excitation beam focal point from the upper surface of the flow path. As demonstrated in the graphs of FIGS. 5 and 6, at a position near the center of the optical path in the flow path, signal intensity was substantially constant even if focal point moved few dozen micrometers in the case of the detection area with the flow path depth of 0.1 mm in contrast to the reaction area with the flow path depth of 0.02 mm. This indicates that the signal can be produced at high reproducibility without being effected by small deviation in the focal point caused, for example, by the vibration of the apparatus, insufficient accuracy in the positioning during the measurement, error from insufficient flatness of the microchip in the case of the detection area where sufficient optical path length is secured in the flow path since any position in the optical path excluding the areas in the vicinity of the upper surface and the lower surface of the flow path can be used.

## Example 2

Comparison of detection sensitivity between the flow paths of different designs: detection of a nucleic acid

A microchip having a standard-type flow path (with a flow path depth in the reaction area of 0.1 mm and the flow path depth in the detection area of 0.1 mm) and the microchip having a flow path according to the present invention (with a flow path depth in the reaction area of 0.02 mm and a flow path depth in the detection area of 0.1 mm) were compared for their detection sensitivity of the target nucleic acid.

The target nucleic acid was detected by a microfluid system as follows. An oligonucleotide modified at its 5' end with biotin and having a sequence complementary to the sequence of the immobilized oligonucleotide was used for the target nucleic acid. More specifically, the flow path of the microchip was filled with BlockAce (product name, manufactured by Snow Brand Milk Products Co.,Ltd.) which had been diluted twice with PBS(-) containing 1 mM EDTA and 0.05% Tween20 (product name by Atlas Powder) (hereinafter referred to as the "washing buffer"), and the microchip was incubated at room temperature (unless otherwise noted, the reaction was hereinafter conducted at room temperature) for 1 hour to thereby block the inner wall of the flow path. Next, the target nucleic acid which had been adjusted to a concentration of 0, 0.2, 1, or 5nM with PBS (-) containing 1%BSA, 1 mM EDTA, and 0.05%Tween20 (product name, Atlas Powder) (hereinafter referred to as the "reaction buffer") was supplied to the different flow paths for 5 minutes, and the flow path was then washed by supplying the washing buffer for 2 minutes. Next, peroxidase (POD)-labeled streptavidin (SA) which had been diluted to 10000 folds with the reaction buffer was supplied for 5 minutes, and the washing buffer was then supplied for 2 minutes. Detection of the target nucleic acid was conducted by thermal lens microscopy (TLM) in which POD activity was detected using SATBlue (product name, manufactured by Dojindo Laboratories) for the substrate. More specifically, while supplying SATBlue (product name, manufactured by Dojindo Laboratories), signal in the depth direction of the flow path was detected by TLM in the detection area in the downstream of the reaction area by setting the excitation beam to a wavelength of 633 nm and the probe beam to a wavelength of 488 nm. The results are shown in Table 1, and in the graph of FIG. 7 wherein the Y axis represents signal intensity of the thermal lens and the X axis represents the concentration of the biotin-labeled oligonucleotide having the complementary sequence.

Table 1

| Concentration of complementary biotin-labeled oligonucleotide (nM) | Signal intensity of a microchip which has a depth of 100 $\mu$m in the reaction area ($\mu$V) | Signal intensity of a microchip which has a depth of 20 $\mu$m in the reaction area ($\mu$V) |
|---|---|---|
| 0 | 27.95 | 28.61 |
| 0.2 | 34.59 | 90.79 |
| 1 | 68.68 | 332.53 |
| 5 | 241.65 | 661.54 |

As demonstrated in Table 1 and FIG. 7, the microchip of the present invention having a smaller reaction area depth of 0.02 mm had a higher detection sensitivity compared to the contrast microchip having a standard-type flow path with a greater reaction area depth of 0.1 mm, indicating a remarkable increase in the detection sensitivity of the present invention.

**Example 3**

Comparison of detection sensitivity between the flow paths of different designs: detection of a protein

A microchip having a standard-type flow path (with a flow path depth in the reaction area of 0.1 mm and the flow path depth in the detection area of 0.1 mm) and the microchip having a flow path according to the present invention (with a flow path depth in the reaction area of 0.02 mm and a flow path depth in the detection area of 0.1 mm) were compared for their detection sensitivity of the target protein.

Detection was conducted by using a surface antigen of hepatitis B virus (HBsAg, manufactured by Meiji Dairies Corporation) for the target protein. More specifically, the flow path of the microchip was filled with BlockAce (product name, manufactured by Snow Brand Milk Products Co.,Ltd.) which had been diluted twice with a washing buffer, and the microchip was incubated at room temperature (unless otherwise noted, all reactions were hereinafter conducted at room temperature) for 1 hour to thereby block the inner wall of the flow path. Next, an anti-HBsAg monoclonal antibody having bonded thereto an oligonucleotide having a sequence complementary to the immobilized oligonucleotide which had been adjusted to a concentration of 50 $\mu$g/mL by the reaction buffer (prepared by the method of Oku et al. (J. Immunol. Methods. Dec 1, 2001; 258(1-2), pp. 73-84.)) was supplied to the flow path for 15 minutes, and the flow path was then washed by supplying the washing buffer for 5 minutes. Next, HBsAg which had been adjusted to a concentration of 0, 0.1, or 0.2 ng/mL by the reaction buffer was supplied to the different flow paths for 15 minutes, and the flow path was washed by supplying the washing buffer for 5 minutes. Next, anti-HBsAg antibody (having a binding site which different from the oligonucleotide-bound antibody) which had been adjusted to a concentration of 1 $\mu$g/mL by the reaction buffer was supplied for 15 minutes, and the flow path was washed by supplying the washing buffer for 5 minutes. POD-labeled streptavidin SA which had been diluted to 10000 folds was then supplied for 15 minutes, and the flow path was washed by supplying the washing buffer for 5 minutes. The target protein was detected by thermal lens microscope (TLM) as in the case of Example 2 by detecting POD activity using SATBlue (product name, manufactured by Dojindo Laboratories) for the substrate.

The results are shown in Table 2, and in the graph of FIG. 8 wherein the Y axis represents signal intensity of the thermal lens and the X axis represents concentration of the HBsAg.

Table 2

| Concentration of HBsAg (ng/mL) | Signal intensity of a microchip which has a depth of 100 $\mu$m in the reaction area ($\mu$V) | Signal intensity of a microchip which has a depth of 20 $\mu$m in the reaction area ($\mu$V) |
|---|---|---|
| 0 | 31 | 29 |
| 0.1 | 31 | 40.255 |
| 0.2 | 34 | 95.15 |

As demonstrated in Table 2 and FIG. 8, the microchip of the present invention having a smaller reaction area depth of 0.02 mm had a higher detection sensitivity compared to the contrast microchip having a standard-type flow path with a greater reaction area depth of 0.1 mm, indicating a remarkable increase in the detection sensitivity of the present invention.

**Comparative Example 1**

Detection sensitivity of HBsAg in ELISA using a microtiter plate

Sandwich ELISA was conducted on a microtiter plate by using the HBsAg antibody combination which was the same as the one used in Example 3. More specifically, 100 μL/well of the anti-HBsAg antibody which had been adjusted to 10 μg/mL with PBS was added to the 96 well microplate, and the reaction was allowed to proceed at room temperature (unless otherwise noted, all reactions were hereinafter conducted at room temperature) for 1 hour. Next, the flow path was washed once with PBS, and 200 μL of BlockAce which had been diluted twice with PBS(-) was supplied for 1 hour for blocking to thereby produce the plate used in the assay. 100 μL of HBsAg which had been adjusted to a concentration of 0, 0.1, or 0.2 ng/mL by the reaction buffer was added to the well, and the reaction was allowed to proceed for 1 hour. After washing the flow path with PBS, 100 μL of the biotin-labeled anti-HBsAg antibody which had been adjusted to a concentration of 1 μg/mL with the reaction buffer was added, and the reaction was allowed to proceed for 1 hour. After washing the flow path with PBS, 100 μL of POD-labeled streptavidin which had been diluted to 10000 folds with the reaction buffer was added, and the reaction was allowed to proceed for 1 hour. After washing, POD activity was determined by color reaction using SATBlue for the substrate by measuring absorption at 660 nm.

The results are shown in Table 3, and in the graph of FIG. 9 wherein the Y axis represents POD activity and the X axis represents the HBsAg concentration.

Table 3

| Concentration of HBsAg (ng/mL) | OD (660 nm) |
|---|---|
| 0 | 0.0495 |
| 0.1 | 0.043 |
| 0.2 | 0.0485 |

As shown in FIG. 9, the microtiter plate method failed to detect the HBsAg even at a concentration of 0.2 ng/mL. This result confirmed the superior sensitivity of the immunoassay system using the microfluid system with the flow path of the present invention as shown in Example 3 compared to the microtiter plate method.

**INDUSTRIAL APPLICABILITY**

The microchip of the present invention is well adapted for use in the assay of a biological substance, and it can be used, for example, in diagnosing various diseases, analyzing genome or protein of an animal, plant, or microorganism, testing the safety of GM foods, and detecting toxic substances in the environment.

**Claims**

1.  A microchip comprising two substrates having at least a flow path formed at the interface between the two substrates, the flow path having a reaction area and a detection area in the downstream of the reaction area, and the flow path at the detection area having a depth which is deeper than the flow path at the reaction area.

2.  A microchip comprising two substrates having at least a flow path formed at the interface between the two substrates, the flow path having a reaction area and a detection area in the downstream of the reaction area, the flow path at the reaction area having a width which is wider than the flow path at the detection area, and the flow path at the detection area having a depth which is deeper than the flow path at the reaction area.

3.  The microchip according to claim 1 or 2 wherein said reaction area has an interface which contributes for the reaction and said interface is at least a part of the interface of the flow path.

4.  The microchip according to claim 1 or 2 wherein the microchip is used for measuring a biological substance, and said interface contributing for the reaction has immobilized thereon an anti-biological substance which is capable of binding to the biological substance to be measured by affinity to thereby capture said biological substance.

5.  The microchip according to claim 4 wherein a nucleic acid is immobilized on the interface of the flow path, and said anti-biological substance is bonded to the nucleic acid.

6.  The microchip according to claim 4 wherein magnetic particles are immobilized on the interface of the flow path by magnetism of a magnet provided in or outside the flow path, and said anti-biological substance is immobilized on the surface of the magnetic particles.

7.  The microchip according to any one of claims 1 to 6 wherein the flow path at the reaction are has a width of 1 $\mu$m to 2 mm.

8.  The microchip according to any one of claims 1 to 6 wherein the flow path at the reaction are has a width of 1 $\mu$m to 500 $\mu$m.

9.  The microchip according to any one of claims 1 to 8 wherein the flow path at the detection are has a depth of 10 $\mu$m to 2 mm.

Fig.1

Fig.2

Fig.3

Fig.4

Fig. 5

Fig. 6

Fig.7

Fig.8

Fig.9

Evaluation of HBsAg detection system using a microtiter plate

## INTERNATIONAL SEARCH REPORT

| | International application No. |
|---|---|
| | PCT/JP2005/021231 |

A. CLASSIFICATION OF SUBJECT MATTER
*C12M1/00*(2006.01), *B01J19/00*(2006.01), *B81B1/00*(2006.01), *C12M1/34*
(2006.01), *G01N37/00*(2006.01)

According to International Patent Classification (IPC) or to both national classification and IPC

B. FIELDS SEARCHED

Minimum documentation searched (classification system followed by classification symbols)
*C12M1/00-3/10*(2006.01)

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 7-506430 A (Trustees of the University of Pennsylvania), 13 July, 1995 (13.07.95), | 1-9 |
| Y | JP 2000-508528 A (The Perkin Elmer Corp.), 11 July, 2000 (11.07.00), | 1-9 |

☐ Further documents are listed in the continuation of Box C.   ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 December, 2005 (13.12.05) | 27 December, 2005 (27.12.05) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**
Information on patent family members

International application No.

PCT/JP2005/021231

| | | |
|---|---|---|
| JP 7-506430 A | 1995.07.13 | JP 7-506256 A |
| | | JP 7-506257 A |
| | | JP 7-506258 A |
| | | JP 7-506431 A |
| | | JP 9-509498 A |
| | | JP 9-511407 A |
| | | JP 3220158 B |
| | | WO 1993/022053 A1 |
| | | WO 1993/022055 A2 |
| | | WO 1993/022054 A1 |
| | | WO 1993/022058 A1 |
| | | WO 1993/022421 A1 |
| | | WO 1996/014933 A1 |
| | | WO 1996/014934 A1 |
| | | WO 1996/015269 A2 |
| | | EP 0637996 A1 |
| | | EP 0637997 A1 |
| | | EP 0637998 A1 |
| | | EP 0637999 A1 |
| | | EP 0639223 A1 |
| | | EP 0739240 A1 |
| | | EP 0739423 A1 |
| | | US 5304487 A1 |
| | | US 5296375 A1 |
| | | US 5635358 A1 |
| | | US 5726026 A1 |
| | | US 5928880 A1 |
| | | US 6184029 B1 |
| | | US 5427946 A1 |
| | | US 5744366 A1 |
| | | US 5498392 A1 |
| | | US 5587128 A1 |
| | | US 5955029 A1 |
| | | US 2003/0199081 A1 |
| | | US 6660517 B1 |
| | | US 5486335 A1 |
| | | US 5637469 A1 |
| | | US 5866345 A1 |
| | | US 6551841 B1 |
| | | US 2003/0129671 A1 |
| | | US 2005/0079634 A1 |
| JP 2000-508528 A | 2000.07.11 | WO 1997/036681 A1 |
| | | EP 0889751 A1 |
| | | US 6124138 A1 |
| | | US 6126899 A1 |
| | | US 2003/0152994 A1 |
| | | US 6825047 B |

Form PCT/ISA/210 (patent family annex) (April 2005)

**EP 1 816 187 A1**

## REFERENCES CITED IN THE DESCRIPTION

### Patent documents cited in the description

- JP 2002365293 A

### Non-patent literature cited in the description

- **SATO et al.** *Analytical chemistry,* 2001, vol. 73, 1213-1218
- *Biosensors and Bioelectronics,* 2004, vol. 19, 1193-1202
- **LAB.** *chip,* 2002, vol. 2, 27-30
- *Anal. Chem.,* 1997, vol. 69, 3451-3457
- *Anal. Chem.,* 1997, vol. 69, 2626
- *Anal. Chem.,* 1997, vol. 69, 4783
- *Analytical Biochemistry,* 2003, vol. 317, 76-84
- **MCDONALD, J. C. et al.** *Electrophoresis,* 2000, vol. 21 (1), 27-40
- **OKU et al.** *J. Immunol. Methods.,* 01 December 2001, vol. 258 (1-2), 73-84

```
                        SEQUENCE LISTING

        <110>  NISSUI PHARMACEUTICAL
               KANAGAWA ACADEMY OF SCIENCE AND TECHNOLOGY

        <120>  Microchip

        <130>  NSM5032PCT

        <160>  1

        <170>  PatentIn version 3.1

        <210>  1
        <211>  20
        <212>  DNA
        <213>  Artificial

        <220>
        <223>  Solid phased oligonucleotide

        <400>  1
        ttgctaaccc agaacactat                                  20
```